# EUROPÄISCHE PATENTSCHRIFT

(11) **EP 4 382 746 B1**
(45) Veröffentlichungstag und Bekanntmachung des Hinweises auf die Patenterteilung: **08.07.2026**
(21) Anmeldenummer: 24172399.8
(22) Anmeldetag: 27.11.2020
(51) Int. Cl.: F04B 1/0452

(54) **PUMPENEINHEIT FÜR MEDIZINISCHE ZWECKE**
PUMP UNIT FOR MEDICAL PURPOSES
UNITÉ DE POMPE À USAGE MÉDICAL

(43) Veröffentlichungstag der Anmeldung: 12.06.2024
(62) Teilanmeldung aus: 20210402.2
(73) Patentinhaber: Erbe Elektromedizin GmbH, 72072 Tübingen (DE)
(72) Erfinder: FEHRENBACHER, Michael, 72108 Rottenburg (DE)
(74) Vertreter: Rüger Abel Patentanwälte PartGmbB

(56) Entgegenhaltungen:
- EP-B1- 2 730 240
- EP-B1- 2 924 285
- DE-A1- 102004 031 673
- DE-A1- 102009 006 042
- US-A- 2 337 510
- US-A1- 2009 126 695
- US-A1- 2019 011 051
- US-B1- 9 377 019

## Beschreibung

Die Erfindung betrifft eine Pumpeneinheit für den medizinischen Einsatz, beispielsweise für die Wasserstrahlchirurgie.

Zu dem genannten Zweck für den medizinischen oder auch anderweitigen Einsatz sind aus der DE 10 2004 031 673 A1, der EP 2 730 240 B1, der DE 103 48 832 A1, der US 2014/0127037 A1, der EP 1 735 030 B1, der EP 2 222 957 B1, der US 8,491,526 B2, der EP 2 758 097 A1 sowie der DE 694 29 306 T2 bekannt. Solche Kolbenpumpen sind häufig mit zwei Zylindern versehen, die gegenläufig angetrieben sind und abwechselnd Fluid, beispielsweise ein flüssiges medizinisches Behandlungsfluid, wie beispielsweise Natriumchlorid-Lösung, ansaugen und weiterfördern. Dazu ist jeder Zylinder typischerweise mit einem Einlassventil und mit einem Auslassventil versehen, die gleich oder unterschiedlich ausgebildet sein können. Dazu offenbart die WO 20133/043881 A1 bei einer Pumpeneinheit mit insgesamt vier Zylinder Ein- und Auslassventile, deren Ventilverschlussglied durch ein gewölbtes flexibles Scheibchen gebildet ist, das in einer Ventilkammer gehalten ist. Durch den wechselnden Fluiddruck gibt es die jeweilige Ein- oder Ausströmöffnung frei oder schließt sie.

Aus der EP 2 711 545 A1 ist dazu eine Pumpeneinheit mit zwei Zylindern bekannt, deren Ein- und Auslassventile jeweils Kugelventile sind. Insbesondere werden Kugelventile verwendet, deren Kugel in einer Ventilkammer lose, d.h. frei beweglich gehalten ist. Die Ventilkugel kann aus Metall oder Kunststoff ausgebildet sein. Die freie Beweglichkeit der Ventilkugeln in der Ventilkammer gestattet ein besonders leichtes Sterilisieren dieser Pumpe durch Sterilisiergas.

Prinzipiell sind auch Ventile mit nicht kugelförmigen und nicht scheibenförmigen Ventilverschlussgliedern bekannt, wie beispielsweise aus der DE 10 2007 052 755 A1, dort für hydraulische Fahrzeugbremsanlagen. Weiter zeigt die EP 1 980 291 A1 ein Ventil für den medizinischen Einsatz mit einem etwa pilzförmigen elastischen Ventilverschlussglied aus einem Gummimaterial. Das Ventilverschlussglied sitzt mit seinem in Schaftrichtung weisenden Rand auf einen Ventilsitz auf und bildet so außen eine Dichtungspaarung.

JP H0835574 A beschreibt eine Pumpeneinheit aufweisend eine von einem Motor angetriebene Pumpe, an die stromabwärts ein Rückschlagventil und ein Wegeventil angeschlossen sind. Das Wegeventil hat ein Pilotventil mit einem Ventilkörper, der an einem Ende ein konisches Ventilverschlussglied bildet. Der Ventilkörper ist durch eine Schließfeder auf einen Pilotventilsitz vorgespannt. Entgegen der Federkraft der Schließfeder kann der Ventilkörper magnetisch vom Pilotventilsitz angehoben werden. Bei geöffnetem Pilotventil wird anschließend ein Hauptventil mit einem topfförmigen Hauptventilglied freigegeben.

Die EP 2 924 285 A1 offenbart ebenfalls ein Ventil mit einem pilzförmigen Ventilverschlussglied, dessen Rand Durchströmungsöffnungen aufweist. Sein Schaft ragt in einen abströmseitigen Strömungskanal, während seine ballig gewölbte, vom Schaft weg weisende Fläche auf einem Ventilsitz aufsitzt. Die Elastizität des etwa tellerförmigen Kopfs des Ventilverschlussglieds generiert die Federkraft, mit der die ballige Dichtfläche des Kopfs an einem Ventilsitz in Form einer den Einströmkanal umgebenden Ringrippe anliegt. Solche Ventile eignen sich prinzipiell gut für den medizinischen Einsatz, wobei sie aber bei sehr hohen Durchströmungsraten an ihre Grenzen kommen können. Dies gilt insbesondere, wenn der Fluidstrom so groß ist, dass das Ventilverschlussglied in den abströmseitigen Kanal verlagert wird und diesen dann verstopft.

Eine weitere bei den vorgenannten Pumpen auftretende Problematik kann sich ergeben, wenn in der Pumpe außer der zu fördernden Flüssigkeit Gas- oder Luftblasen verbleiben. Die Pumpen sollen möglichst leicht entlüftbar sein. Außerdem wird ein einfacher und leicht steril zu haltender Aufbau gewünscht.

Es ist Aufgabe der Erfindung, wenigstens einem der vorgenannten Probleme abzuhelfen.

Diese Aufgabe wird mit der Pumpeneinheit nach Anspruch 1 gelöst:
Die erfindungsgemäße Pumpeneinheit weist mindestens einen, vorzugsweise zwei, Zylinder auf, in denen jeweils ein Kolben verschiebbar gelagert ist. Der Kolben ist gegen die Zylinderwandung abgedichtet und weist dazu beispielsweise ein aus einem elastischen Material gebildeten Kopf auf. An seinem von diesem Kopf abliegenden Ende ragt eine mit dem Kolben verbundene Kolbenstange aus dem Zylinder heraus und ist an ihrem freien Ende mit einer Kupplungseinrichtung versehen, mit der sie vorzugsweise formschlüssig mit einer Antriebseinrichtung kuppelbar ist.

Der Zylinder steht mit einem ersten als Einlasskanal dienenden Kanal in Verbindung, in dem ein erstes Ventil angeordnet ist, das als Einlassventil dient. Ebenso steht der Zylinder mit einem zweiten als Auslasskanal dienenden Kanal in Verbindung, in dem ein zweites Ventil angeordnet ist, das als Auslassventil dient. Wenigstens eines der beiden Ventile kann pilz- oder schirmförmig ausgebildet sein, d.h. es weist einen etwa scheibenförmigen Kopfabschnitt und einen sich von der Scheibenmitte weg erstreckenden Schaft auf. Das Ventilverschlussglied ist in dem Ventil mit Spiel oder spielfrei gelagert. Es kann sich einerseits mit einer von dem Schaft weg weisenden Stirnfläche an dem Ventilsitz und andererseits mit dem freien Ende des Schafts an einem dem Ventilsitz gegenüberliegenden Widerlager spielfrei abstützend angeordnet sein. Das Ventilverschlussglied kann dabei einstückig aus einem elastischen Material, beispielsweise einem gummiartigen Material ausgebildet sein. Das Ventilverschlussglied kann den Ventilsitz und somit den Fluiddurchgang freigeben, indem sich der Ventilkopf etwas von dem Ventilsitz weg wölbt und/oder in dem sich der Schaft des Ventilverschlussglieds etwas komprimiert. Damit kann sich die dem Ventilsitz zugewandte Dichtfläche des Ventilverschlussglieds insgesamt etwas von dem Ventilsitz weg bewegen. Auf die Weise wird der Durchflusswiderstand des Ventils in Offenstellung reduziert. Nach diesem Konzept, bei dem der Schaft des Ventilverschlussglieds zum Lagern und Zentrieren des Ventilverschlussglieds dient, ist zudem ausgeschlossen, dass das Ventilverschlussglied bei hohen Strömungsgeschwindigkeiten von der Flüssigkeit mitgerissen und in den abströmseitigen Kanal getrieben wird.

Vorzugsweise ist das Widerlager, an dem sich der Schaftabschnitt stirnseitig abstützt, Teil einer nicht von dem Fluid durchströmten Stützanordnung, die den Schaftabschnitt umgibt. Das stirnseitige Ende dieser Stützanordnung kann zugleich eine Anlagefläche für den Kopfabschnitt des Ventilverschlussglieds bilden, sodass dieses in Offenstellung eine definierte Axialposition einnimmt und sicher gehalten bleibt. Die Stützanordnung weist eine vorzugsweise fünfseitig (am Boden und am Umfang) geschlossene Tasche auf, in der der Schaftabschnitt sitzt. Die Stützanordnung kann mit dem Kanal, in dem sie angeordnet ist, einen ringförmigen Strömungsquerschnitt festlegen. Dieses Konzept gestattet eine umlenkungsarme Strömungsführung und eine sichere Fassung oder Lagerung des Ventilverschlussglieds. Dies gilt auch, wenn das Ventilverschlussglied bei bestimmten in dem Ventil mit Spiel gehalten ist. Bei solchen Ausführungsformen ist auch sichergestellt, dass das Ventilverschlussglied, nachdem sein Schaft in der Stützanordnung gefangen ist, nicht unerwünscht disloziert und schlimmstenfalls sogar in den Kanal gespült werden kann.

Unabhängig von der konkreten Ausbildung des Ventils ist es vorteilhaft, zwei oder mehrere Zylinder der Pumpeneinheit in Gebrauch horizontal und vertikal übereinander anzuordnen. Dies erleichtert die Entlüftung der Pumpeneinheit. Das gilt insbesondere, weil bei jedem Zylinder das zweite, als Auslassventil dienende Ventil oberhalb des ersten, als Einlassventil dienenden Ventils angeordnet ist. Dabei geht der Auslasskanal möglichst in der Nähe der Zylinderwand aus dem Zylinder ab. Somit können Luftblasen sicher aus dem Zylinder ausgetrieben werden, sodass der erzeugte Flüssigkeitsstrom präzise der Vorgabe durch die Pumpe folgt.

Weiterhin ist es sowohl bei Pumpeneinheiten gemäß Anspruch 1 wie auch bei Pumpeneinheiten generell vorteilhaft, wenn der Zylinder einen ersten Abschnitt und einen zweiten Abschnitt aufweist, die unterschiedliche Durchmesser haben. Der erste, den Ventilen nahe Abschnitt weist dabei vorzugsweise einen geringeren Durchmesser auf. Der zweite, den Ventilen ferne Abschnitt weist vorzugsweise einen größeren Durchmesser auf. Die axiale Länge des zweiten Abschnitts ist vorzugsweise wenigstens so groß, wie die axiale Länge des ersten Abschnitts. An dem Kolben sind vorzugsweise zwei Dichtungen vorgesehen, wobei die erste Dichtung dem ersten Abschnitt und die zweite Dichtung dem zweiten Abschnitt zugeordnet ist. Der Abstand zwischen der ersten und der zweiten Dichtung ist dabei vorzugsweise so groß wie die Länge des ersten Abschnitts oder größer. Die erste Dichtung dient dabei der Abdichtung des sterilen Pumpvolumens, das der Kolben in dem Zylinder abgrenzt. Die zweite Dichtung dient der Abdichtung der Pumpeneinheit nach außen und verhindert das Eindringen nicht steriler Verschmutzungen in die Pumpeneinheit. Die beiden Dichtungen dichten jeweils an der Wandung des Zylinders, d.h. an seiner zylindrischen Innenfläche ab. Balgdichtungen oder dergleichen, die sonst zur Abdichtung der Kolbenstange gegen den Zylinder dienen, können entfallen.

Weitere Ausführungsformen ergeben sich aus der Zeichnung, der Beschreibung oder Ansprüchen. Es zeigen:
Figur 1 eine Pumpeneinheit in perspektivischer teilweiser aufgebrochener Gesamtansicht,
Figur 2 einen Zylinder der Pumpeinheit nach Figur 1 mit Ein- und Auslasskanal, in längsgeschnittener Darstellung,
Figur 3 ein Ventil der Pumpeneinheit nach Figur 1 und 2, in vergrößerter längsgeschnittener Darstellung, in geschlossener Position,
Figur 4 das Ventil nach Figur 3 in Offenstellung
Figur 5 die Pumpeinheit nach Figur 1, in schematisierter Stirnansicht mit Verdeutlichung ihrer Kanalführung,
Figur 6 einen Zylinder der Pumpeneinheit nach Figur 1 bis 5, in längsgeschnittener Darstellung,
Figur 7 eine Pumpeneinheit ähnlich Figur 2 mit modifizierter Kanalanordnung und
Figur 8 ein Ventil ähnlich Figur 3 und 4, in abgewandelter Ausführung.

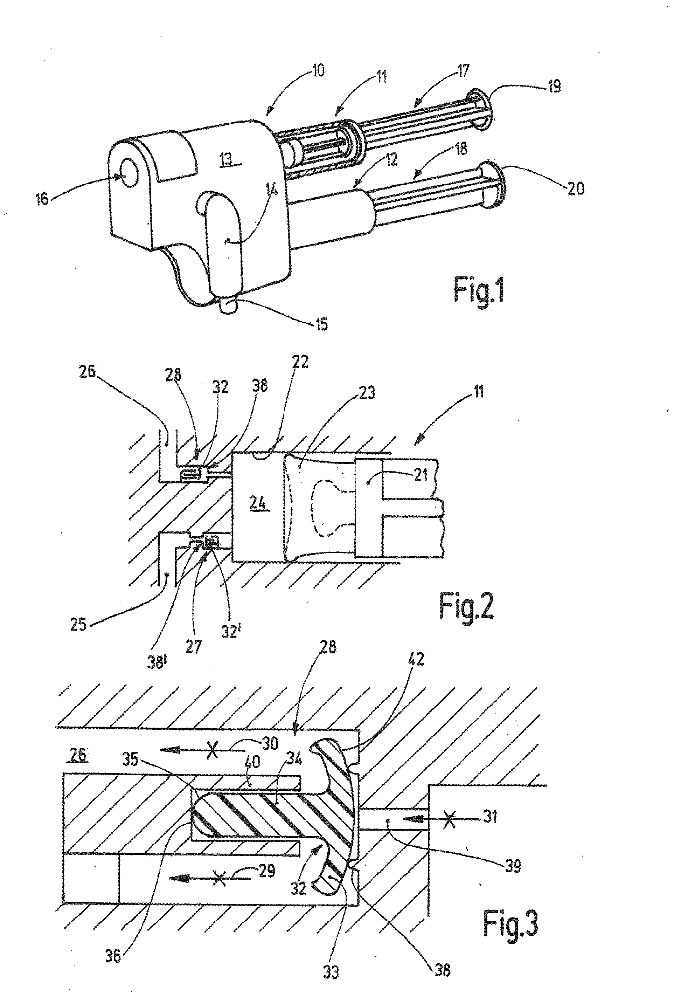

In Figur 1 ist eine Pumpeneinheit 10 veranschaulicht, die als sterilisiertes Medizinprodukt beispielsweise in eine entsprechende Aufnahme eines Geräts eingesetzt werden kann, um ein Fluid, insbesondere eine Flüssigkeit, zu fördern. Dazu weist die Pumpeneinheit 10 zwei Zylinder 11, 12 sowie einen Zylinderkopf 13 auf, von dem sich die Zylinder 11, 12 weg erstrecken. An dem Zylinderkopf 13 ist ein Anschlussstück 14 angeordnet, über das den beiden Zylindern 11, 12 Fluid zuführbar ist und an das, zum Beispiel an einem Ansatz 15, ein zu einem Flüssigkeitsbehälter, beispielsweise einem Beutel, führender Schlauch anschließbar ist. An dem Zylinderkopf 13 ist außerdem ein ausgangsseitiger Anschluss 16 vorgesehen, an den ein Instrument anschließbar ist, das von der Pumpeneinheit 10 mit Fluid zu speisen ist.

Aus den Zylindern 11, 12 ragen Kolbenstangen 17, 18 heraus, die an ihren freien Enden jeweils mit Koppelmitteln 19, 20 versehen sind. Die Koppelmittel 19, 20 gestatten den Anschluss der Kolbenstangen 17, 18 an geeignete Antriebsvorrichtungen, um die Kolbenstangen 17, 18 gezielt und vorzugsweise gegenläufig in den Zylindern 19, 20 axial zu bewegen.

Das oben erwähnte Anschlussstück 14 ist beispielsweise aus flexiblem Material ausgebildet und von dem Zylinderkopf 13 abnehmbar. Auf diese Weise können beide Zylinder 11, 12 mit einem über den Ansatz 15 zugeführten Fluid gleichermaßen versorgt werden. Es ist aber auch möglich, die beiden Zylinder 11, 12 mit unterschiedlichen Fluidquellen zu verbinden, um an dem Anschluss 16 ein Flüssigkeitsgemisch oder von Zeit zu Zeit unterschiedliche Flüssigkeiten, zum Beispiel Spülflüssigkeit und Behandlungsflüssigkeit abzugeben.

Die beiden Zylinder 11, 12 sind vorzugsweise gleich ausgebildet. Die nachfolgende Beschreibung des Zylinders 11 gilt somit entsprechend auch für den Zylinder 12.

Wie in Figur 2 veranschaulicht, ist in dem Zylinder 11 ein Kolben 21 gelagert, der einen gegen die Wandung 22 des Zylinders 11 abgedichteten Kopf 23 aufweist. Der Kopf 23 grenzt in dem Zylinder 11 zusammen mit der Wandung 22 ein Arbeitsvolumen 24 ab, das mit einem ersten, als Einlasskanal dienenden Kanal 25 sowie mit einem zweiten, als Auslasskanal dienenden Kanal 26 in Verbindung steht. In dem ersten Kanal 25 ist ein erstes, als Einlassventil dienendes Ventil 27 angeordnet. In dem zweiten Kanal 26 ist ein zweites, als Auslassventil dienendes Ventil 28 angeordnet.

Eine Besonderheit der erfindungsgemäßen Pumpeneinheit 10 liegt in der Ausbildung mindestens eines der Ventile 27, 28. Die beiden Ventile 27, 28 können gleich ausgebildet sein. Es handelt sich um selbstgesteuerte (differenzdruckgesteuerte) Ventile, so genannte Rückschlagventile.

Die nachstehende Beschreibung des Ventils 28 gilt entsprechend für das Ventil 27:
Es wird zunächst auf Figur 3 Bezug genommen, in der das Ventil 28 in Ruheposition, d.h. geschlossen veranschaulicht ist. Entsprechende, die Strömungsrichtung andeutende, Pfeile 29, 30, 31 sind deswegen durchkreuzt veranschaulicht, denn es findet kein Fluidfluss statt.

Das Ventil 28 weist ein Ventilverschlussglied 32 auf, das, wie der Längsschnitt in Figur 3 erkennen lässt, etwa pilzförmig auch vorzugsweise einteilig aus einem elastischen Material ausgebildet ist. Von einem ungefähr scheibenförmigen, vorzugsweise leicht gewölbten Kopf 33 streckt sich ein vorzugsweise gerader Schaft 34 weg. Der Schaft 34 liegt mit seinem distalen Ende 35 an einem Widerlager 36 an, das in dem Kanal 26 ortsfest angeordnet ist.

Der Kopf 33 weist eine von dem Schaft 34 weg weisende vorzugsweise leicht gewölbte Dichtfläche 42 auf, die an einem Ventilsitz 38 anliegt. Der Ventilsitz 38 wird beispiels- und vorzugsweise durch einen ringförmigen, kreisförmigen Vorsprung gebildet, der den zuströmseitigen Teil 39 des Kanals 26 umgibt. Außerhalb des Ventilsitzes kann der Kopf 33 Ausnehmungen aufweisen.

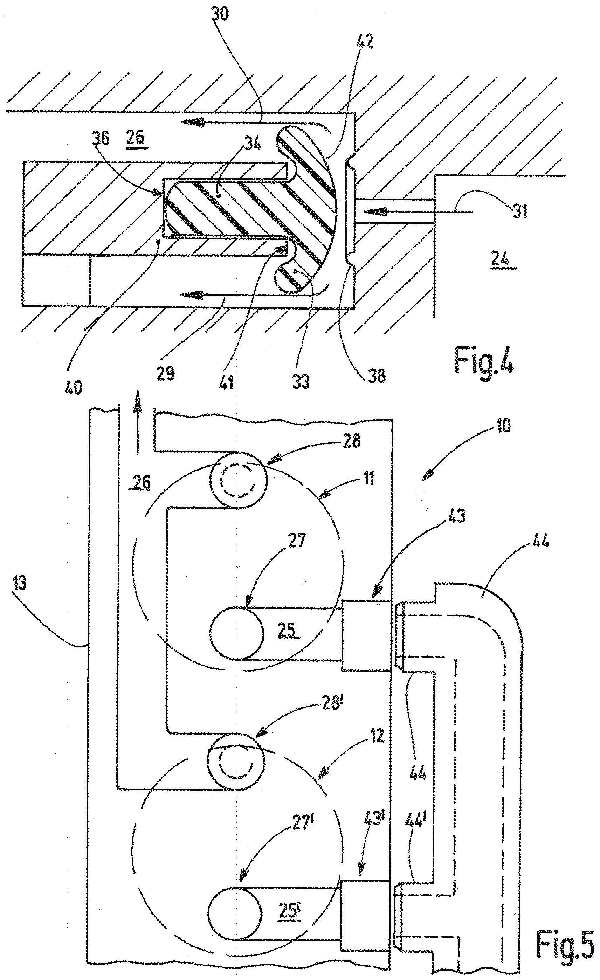

Das Ventil 28 arbeitet wie folgt:
Wie in Figur 3 veranschaulicht ist das Ventilverschlussglied 32 zwischen einerseits dem Ventilsitz 38 und andererseits dem Widerlager 36 mit oder ohne Spiel gefangen. Im Ausführungsbeispiel nach den Figuren 3 und 4 ist das Ventilverschlussglied 32 zwischen dem Widerlager 36 und dem Ventilsitz 38 spielfrei gehalten. Der Kanal 26 ist somit abgesperrt. Fluid fließt nicht in der durch die Pfeile 29, 30, 31 bezeichneten Strömungsrichtung. Zur Verdeutlichung dieses Sachverhalts sind die Pfeile 29 bis 31 durchkreuzt.

Wenn nun ein ausreichender Druckunterschied entsteht, indem der Kolben das Arbeitsvolumen 24 verringert, drängt das in dem Arbeitsvolumen 24 vorhandene Fluid den Kopf 33 von dem Ventilsitz 38 weg, wobei der Kopf 33 etwas deformiert werden kann, indem sich sein Rand zu dem ringförmigen Bereich des Kanals 26 hin wölbt. Der Schaft 34 ist dabei sicher in einer Stützstruktur 40 gehalten, die in dem Kanal 26 ortsfest angeordnet ist. Das Widerlager 36 kann dabei den Boden einer taschenförmigen oder sackbohrungsartigen Vertiefung bilden, die den Schaft 34 aufnimmt und das dem Kopf 33 zugewandte Ende der ansonsten zum Beispiel zapfenartigen Stützstruktur 40 bildet. Das dem Kopf 33 zugewandte stirnseitige Ende der Stützstruktur 40 wird vorzugsweise durch eine Stirnfläche 41 gebildet an die sich der Kopf 33 anlegen kann, wenn der Schaft 34 komprimiert ist.

Wie in Figur 4 dargestellt, hebt die gewölbte dem Ventilsitz 38 zugewandte Dichtfläche 42 des Kopfs 33 von dem Ventilsitz 38 ab und gibt den Strömungsweg für ein Fluid, insbesondere eine Flüssigkeit frei. Der Schaft 34 kann dabei etwas komprimiert und der Kopf 33 kann etwas deformiert sein. Die Pfeile 29, 30 zeigen den Verlauf der Strömung an, die den ringförmigen Strömungsquerschnitt des Kanals 26 durchsetzt und somit die Stützstruktur außen umströmt. Die durch die Pfeile 29, 30 angedeutete Strömung fließt von dem Kopf 33 in den Ringquerschnitt des Kanals 26 und dabei jedenfalls nicht radial nach innen zu dem Schaft 34.

Weil der Schaft 34 in der Stützstruktur 40 sicher gehalten ist und das Fluid nicht entlang des Schafts 34 strömt, kann das Ventilverschlussglied 32 durch die Strömung nicht aus seiner Fassung heraus in den Kanal 26 mitgerissen werden und bleibt somit auch bei hohen Strömungsgeschwindigkeiten funktionsfähig. Andererseits spricht es schon auf geringe Druckunterschiede an und gestattet somit auch die Einstellung sehr niedriger Effektstärken, bei denen mit sehr niedrigen Strömungsgeschwindigkeiten gearbeitet werden muss. Außerdem eignet sich das insoweit beschriebene Ventil auch als Einlassventil (das Ventil 27 in Figur 2). Wegen der geringen zum Öffnen des Ventils nötigen Druckdifferenz ist es als Ansaugventil sehr gut geeignet und in der Lage, Flüssigkeit auch dann anzusaugen, wenn der Zylinder 11 oder 12 zunächst nur mit Luft gefüllt ist.

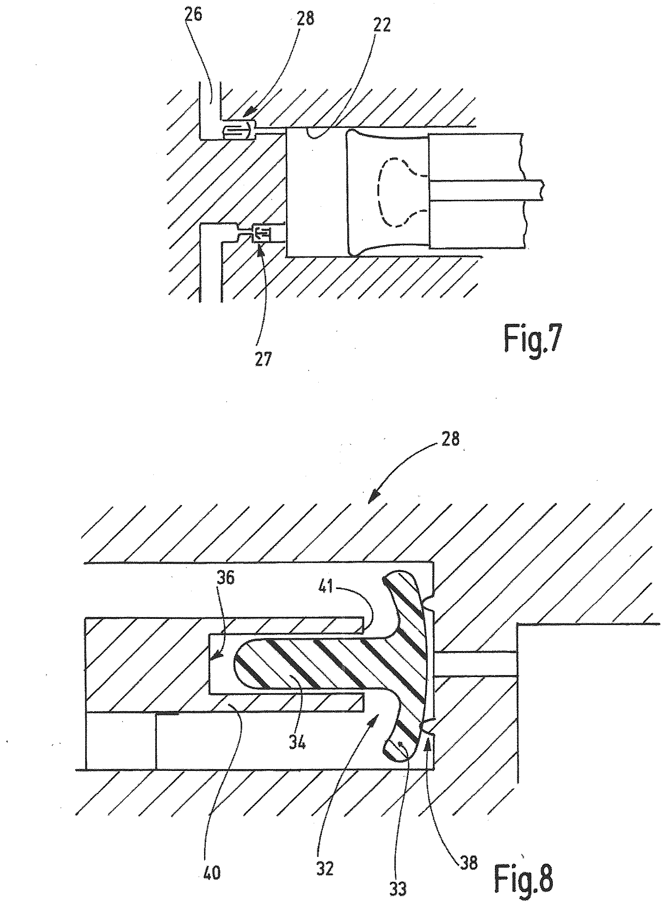

Unabhängig von der konkreten Ausbildung des Ventils 27 oder 28 sind die Ventile 27, 28 vorzugsweise, wie in Figur 5 veranschaulicht, übereinander angeordnet. Das obere Ventil 28 dient dabei als Auslassventil während das untere Ventil 27 vorzugsweise als Einlassventil dient. Entsprechend weist der untere Zylinder 12 ein erstes Ventil 27' als Einlassventil und ein zweites Ventil 28' als Auslassventil auf. Der Kanal 26 ist dabei mit beiden Auslassventilen 28, 28' verbunden, die an den jeweiligen Zylinder 11, 12 oben angeschlossen sind. Wie Figur 7 insbesondere veranschaulicht, geht der Kanal 26 vorzugsweise in unmittelbarer Nachbarschaft zu der Wandung 22 aus dem jeweiligen Zylinder 11 oder 12 ab. Die Position des ersten Ventils 27 ist hingegen nachrangig.

Nach diesem Konzept lässt sich die Pumpeneinheit 10 besonders leicht entlüften. In den Zylindern 11 und 12 enthaltene Luft wird durch eine Pumpbewegung des jeweiligen Kolbens leicht entfernt und durch den Kanal 26 herausgedrängt.

Während die beiden Ventile 28, 28' in den gleichen Kanal 26 münden, der in dem Zylinderkopf 13 fest ausgebildet ist, ist der zum Flüssigkeitseinlass dienende erste Kanal 25 über eine Anschlussstruktur 43 z.B. in Gestalt einer Fluidsteckbuchse separat zugänglich. Dies gilt unabhängig von der sonstigen Ausgestaltung der Pumpeneinheit 10. Die Anschlussstruktur 43 kann zum Beispiel eine Öffnung sein, in die ein Fluidstecker einsetzbar ist. Ebenso kann der zu dem ersten Ventil 27' des zweiten Zylinders 12 führende erste Kanal 25' an einer Anschlussstruktur 43' z.B. in Gestalt einer Fluidsteckbuchse münden. Damit können an die beiden Zylinder 11, 12 bzw. Kanäle 25, 25' jeweils separat Flüssigkeitsvorratsbehälter angeschlossen werden, um die beiden Zylinder 11, 12 mit unterschiedlichen Flüssigkeiten zu füllen und somit ein und dasselbe Instrument mit unterschiedlichen Flüssigleiten versorgen zu können. Es ist jedoch auch möglich, wie es Figur 1 und 5 im Verbund zeigen, ein Verzweigungsstück 44 beispielsweise aus einem flexiblen Kunststoff auszubilden, das einen von dem Ansatz 15 ausgehenden, sich intern verzweigenden Kanal aufweist, der an zwei Steckern 44, 44' mündet, die in die Anschlussstrukturen 43, 43' einsetzbar sind.

Figur 8 veranschaulicht eine Abwandlung des Ventils 28 (oder 27) unabhängig von der sonstigen Ausbildung der Pumpeneinheit 10. Diese kann, wie vorstehend beschrieben ausgebildet sein oder auch davon abweichen. Im Unterschied zu dem vorbeschriebenen Ventil 28 weist das in Figur 8 veranschaulichte Ventil 28 eine Stützstruktur 40 auf, bei der der Abstand zwischen dem Widerlager 36 und dem Ventilsitz 38 größer ist als die in gleicher Richtung zu messende Länge des Ventilverschlussglieds 32. Damit ist die Position des Ventilverschlussglieds 32 in drucklosem Zustand unbestimmt. Jedoch reichen kleinste Flüssigkeitsbewegungen, um den Kopf 33 des Ventilverschlussglieds 32 an den Ventilsitz 38 anzulegen oder von diesem wegzutreiben. Der große schirmartige Kopf 33 bewirkt dabei ein sicheres Überführen des Ventilverschlussglieds 32 in Schließstellung oder Offenstellung je nach Strömungsrichtung.

Auch bei dieser Ausführungsform des Ventils 28 ist der Schaft 34 des Ventilverschlussglieds 32 sicher in der Stützstruktur 40 gefangen und wiederum bildet die den Kopf 33 zugewandte Stirnfläche der Stützstruktur 40 eine Stützfläche 41, an der sich der Kopf 33 abstützen kann, wenn das Ventil 28 in Offenstellung geht. Das dargestellte Ventil 28 in Figur 8 dient als Auslassventil. Es kann jedoch in dieser Struktur ebenso gut als Einlassventil Anwendung finden. Es ist möglich, beide Ventile 27, 28 jeweils gleich nach dem Beispiel von Figur 3 oder 4 auszubilden. Beide Ventile 27, 28 können, falls gewünscht, auch gleich nach dem Beispiel der Figur 8 ausgebildet werden. Darüber hinaus ist es möglich, ein Ventil, beispielsweise das erste Ventil 27 nach dem Beispiel der Figur 8 und das zweite Ventil 28 nach dem Beispiel von Figur 3 und 4 auszubilden oder umgekehrt.

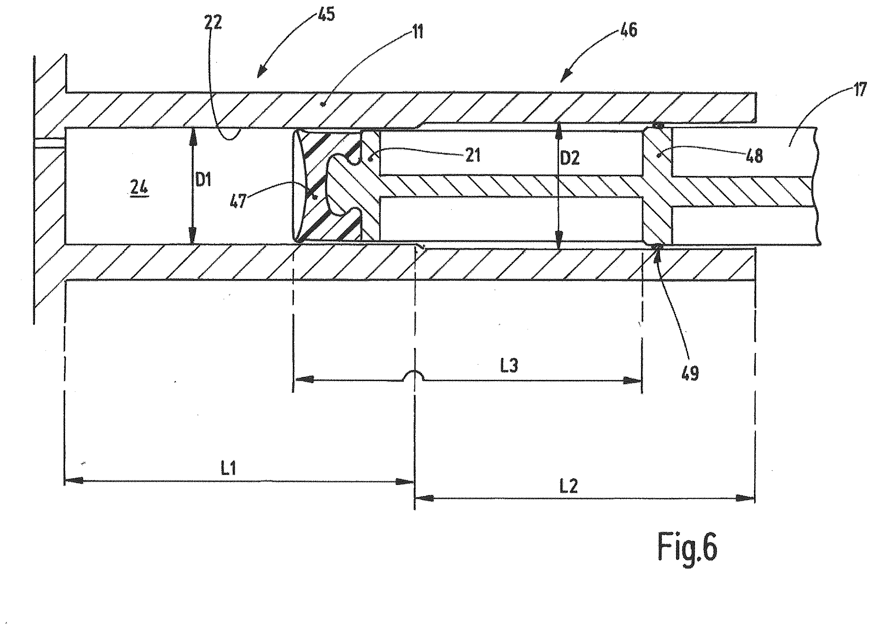

Figur 6 veranschaulicht die Ausbildung des Kolbens 21 und des zugehörigen Zylinders 11. Diese Gestaltung kann unabhängig von der Beschaffenheit der Ventile 27, 28 oder sonstiger Details der Pumpeinheit 10 an einer ein- oder mehrzylindrigen Pumpeneinheit gewählt werden. Der Zylinder 11 weist einen ersten Abschnitt 45 mit einer Länge L1 auf (Pumpabschnitt), in dem er einen Durchmesser D1 aufweist. An den ersten Abschnitt 45 schließt sich ein zweiter Abschnitt 46 mit einer Länge L2 und einem Durchmesser D2 an (Dichtabschnitt). Der Durchmesser D2 ist dabei vorzugsweise wenigstens etwas größer als der Durchmesser D1. Zwischen beiden Abschnitten 45 und 46 kann ein kurzer sich konisch verjüngender Abschnitt vorgesehen sein, in dem die Wandung 22 von dem Durchmesser D2 auf den Durchmesser D1 übergeht. Die Länge des zweiten Abschnitts L2 ist dabei vorzugsweise wenigstens so groß wie die Länge L1.

Der Kolben 21 weist kopfseitig einen Dichtungskörper 47 auf, der mit der Wandung 22 abdichtet und dazu eine gerundete radial nach außen gerichtete Lippe aufweist. Der Kolben 21 weist in einem sich von dem Dichtungskörper 47 weg erstreckenden Bereich einen unrunden beispielsweise kreuzförmigen Abschnitt auf. In einem Abstand L3 von der Stirnseite des Dichtungskörpers 47 gemessen weist der Kolben 21 einen scheibenförmigen Abschnitt 48 auf, an den sich die Kolbenstange 17 anschließt. Der Scheibenabschnitt 48 ist an seinem Außenumfang mit einer Dichtung 49, beispielsweise einer O-Ring-Dichtung, einer Lippendichtung oder dergleichen versehen, die mit der Zylinderwandung 22 abdichtet. Bei einer gebrauchsgemäßen Hin- und Her-Bewegung des Kolbens 21, d.h. beim Durchlaufen eines vollständigen Pumpenhubs, durchstreift der Dichtungskörper 47 den ersten Abschnitt 45. Mit anderen Worten, der Pumpenhub des Kolbens 21 ist geringer als die Länge L1. Damit bewegt sich der Scheibenabschnitt 48 einschließlich seiner Dichtung 49 ausschließlich in dem zweiten Bereich 46. Während der Kolben 21 mit dem Dichtungskörper 47 das sterile Arbeitsvolumen 24 begrenzt, hält die Dichtung 49 des Scheibenabschnitts 48 Umgebungskeime sicher von dem zwischen dem Dichtungskörper 47 und der Dichtung 49 eingeschlossenen Bereich fern, sodass die Sterilität der Wandung 22 in dem ersten Abschnitt 45 sichergestellt werden kann.

Die erfindungsgemäße Pumpeneinheit 10 weist wenigstens ein verbessertes Ventil 28 (oder 27) auf, in dem ein nicht kugelförmiges Ventilverschlussglied 32 in einer Stützstruktur 40 sicher gehalten und in der die Stützstruktur 40 außen umströmt ist. Ein Schaft 34 des Ventilverschlussglieds 32 ist in einer taschenförmigen Aufnahme der Stützstruktur 40 gefangen, sodass das Ventilverschlussglied 32 auch bei großer Durchströmung sicher am Platz gehalten ist. Die Stützstruktur 40 ist dazu lediglich außen umströmt. Der Schaft 34 ist von einer nicht durchströmten Fassung aufgenommen, die in der Stützstruktur 40, beispielsweise in Gestalt eine Sackbohrung, ausgebildet ist. Vorzugsweise ist die Pumpeneinheit mit zwei Zylindern 11, 12 versehen, die in Gebrauch liegend, dabei jedoch vertikal übereinander angeordnet sind. Insbesondere aber sind das erste Ventil 27 und das zweite Ventil 28 vorzugsweise übereinander angeordnet. Wiederum vorzugsweise ist das dem Auslasskanal zugeordnete zweite Ventil 28 in unmittelbarer Nachbarschaft der Zylinderwandung 22 am vertikal höchsten Punkt der Zylinderwandung 22 angeordnet. Dies erleichtert die Entlüftung der Pumpeneinheit 10.

### Bezugszeichen:

- 10: Pumpeneinheit
- 11: oberer Zylinder
- 12: unterer Zylinder
- 13: Zylinderkopf
- 14: Anschlussstück
- 15: Ansatz
- 16: Anschluss
- 17: obere Kolbenstange
- 18: untere Kolbenstange
- 19: oberes Koppelmittel
- 20: unteres Koppelmittel
- 21: Kolben
- 22: Wandung des Zylinders 11
- 23: Kopf des Kolbens 21
- 24: Arbeitsvolumen
- 25, 25': erster Kanal / Einlasskanal
- 26: zweiter Kanal / Auslasskanal
- 27, 27': erstes Ventil / Einlassventil
- 28, 28': zweites Ventil / Auslassventil
- 29 - 31: Pfeile zur Veranschaulichung der Strömungsrichtung
- 32: Ventilverschlussglied
- 33: Kopf des Ventilverschlussgliedes 32
- 34: Schaft des Ventilverschlussgliedes 32
- 35: Ende des Ventilverschlussgliedes 32
- 36: Widerlager
- 38, 38': Ventilsitz (rippenartiger ringförmiger Vorsprung)
- 39: Teil des Kanals 26
- 40: Stützstruktur
- 41: Stützfläche
- 42: Dichtfläche
- 43, 43': Anschlussstruktur
- 44, 44': Stecker
- 45: erster Abschnitt des Zylinders 11
- L1: Länge des ersten Abschnitts 45 des Zylinders 11
- D1: Durchmesser des ersten Abschnitts 45
- 46: zweiter Abschnitt des Zylinders 11
- L2: Länge des zweiten Abschnitts 46 des Zylinders 11
- D2: Durchmesser des zweiten Abschnitts 46
- 47: Dichtungskörper
- 48: Scheibenabschnitt
- 49: Dichtung

## Patentansprüche

1. Medizinische Pumpeneinheit (10) für die Wasserstrahlchirurgie,
mit mindestens einem Zylinder (11) mit einem darin verschiebbar gelagerten Kolben (21),
mit einem mit dem Zylinder (11) verbundenen ersten Kanal (25), in dem ein erstes Ventil (27) mit einem ersten, einem ersten Ventilsitz (38') zugeordneten Ventilverschlussglied (32') angeordnet ist,
mit einem mit dem Zylinder (11) verbundenen zweiten Kanal (26), in dem ein zweites Ventil (28) mit einem zweiten, einem zweiten Ventilsitz (38) zugeordneten Ventilverschlussglied (32) angeordnet ist,
**dadurch gekennzeichnet,**
**dass** das erste Ventil (27) als Einlassventil dient und das zweite Ventil (28) als Auslassventil dient, die übereinander angeordnet sind, wobei das zweite Ventil (28) über dem ersten Ventil (27) angeordnet ist.

2. Pumpeneinheit nach Anspruch 1, **dadurch gekennzeichnet, dass** die Ventile (27, 28) differenzdruckgesteuerte Ventile sind.

3. Pumpeneinheit nach Anspruch 1 oder 2, **dadurch gekennzeichnet**, der Kolben gegen eine Zylinderwandung (22) des mindestens einen Zylinders (11) abgedichtet ist, und dass das zweite Ventil (28) in unmittelbarer Nachbarschaft der Zylinderwandung (22) am vertikal höchsten Punkt der Zylinderwandung (22) angeordnet ist.

4. Pumpeneinheit nach einem der vorhergehenden Ansprüche,
**dadurch gekennzeichnet, dass** wenigstens eines der Ventilverschlussglieder (32) nahtlos einstückig aus einem elastischen Material ausgebildet ist.

5. Pumpeneinheit nach einem der vorstehenden Ansprüche,
**dadurch gekennzeichnet, dass** wenigstens eines der Ventilverschlussglieder (32) eine gewölbte, dem Ventilsitz (38) zugewandte Dichtfläche (42) aufweist.

6. Pumpeneinheit nach einem der vorstehenden Ansprüche,
**dadurch gekennzeichnet, dass** wenigstens einer der Ventilsitze (38) ein ringförmiger rippenartiger Vorsprung ist, der eine Zuströmöffnung umgibt.

7. Pumpeneinheit nach einem der vorstehenden Ansprüche,
**dadurch gekennzeichnet, dass** wenigstens eines der Ventilverschlussglieder (32, 32') einen elastischen Schaftabschnitt (34) aufweist, mit dem es in einer dem Ventilsitz (38, 38') gegenüberliegenden nicht durchströmten Stützstruktur (40) gefasst angeordnet ist, und dass das wenigstens eines der Ventilverschlussglieder (32) einen Kopfabschnitt (33) aufweist, von dem sich der elastische Schaftabschnitt (34) weg erstreckt.

8. Pumpeneinheit nach Anspruch 7, **dadurch gekennzeichnet, dass** der Schaftabschnitt (34) gerade und druckelastisch ausgebildet ist.

9. Pumpeneinheit nach Anspruch 7 oder 8, **dadurch gekennzeichnet, dass** wenigstens eines der Ventilverschlussglieder (32) zwischen einem an seiner Stützstruktur (40) ausgebildeten Widerlager (36) und dem Ventilsitz (38) spielfrei gehalten ist.

10. Pumpeneinheit nach Anspruch 9, **dadurch gekennzeichnet, dass** die Stützstruktur (40) den Schaftabschnitt (34) umgebend angeordnet ist.

11. Pumpeneinheit nach Anspruch 10, **dadurch gekennzeichnet, dass** die Stützstruktur (40) in dem Kanal (26) angeordnet ist und mit dessen Wandung einen ringförmigen Strömungsquerschnitt festlegt.

12. Pumpeneinheit nach einem der vorstehenden Ansprüche,
**dadurch gekennzeichnet, dass** sie zwei Zylinder (11, 12) aufweist, die jeweils horizontal liegend angeordnet sind.

13. Pumpeneinheit nach Anspruch 12, **dadurch gekennzeichnet, dass** die beiden Zylinder (11, 12) vertikal übereinander angeordnet sind.

14. Pumpeneinheit nach einem der vorstehenden Ansprüche,
**dadurch gekennzeichnet, dass** der Zylinder (11, 12) einen ersten Abschnitt (45) und einen zweiten Abschnitt (46) aufweist, wobei der zweite Abschnitt (46) einen Durchmesser (D2) aufweist, der größer ist als der Durchmesser (D1) des ersten Abschnitts (45).

15. Pumpeneinheit nach Anspruch 14, **dadurch gekennzeichnet, dass** der Kolben (21) sowohl in dem ersten Abschnitt (45) des Zylinders (11, 12) als auch in dem zweiten Abschnitt (46) des Zylinders (11, 12) jeweils mit einer Dichtung (47, 49) versehen ist, die den Kolben (21) gegen den Zylinder (11, 12) abdichtet.

16. Pumpeneinheit nach Anspruch 15, **dadurch gekennzeichnet, dass** der erste Abschnitt (45) und der zweite Abschnitt (46) des Zylinders (11, 12) jeweils wenigstens so lang sind, wie der maximale Pumpenhub des Kolbens (21).

## Claims

1. Medical pump unit (10) for water-jet surgery,
having at least one cylinder (11) with a piston (21) displaceably supported therein,
having a first channel (25) connected with the cylinder (11), wherein in the first channel (25) a first valve (27) is arranged having a first valve closing member (32') assigned to a first valve seat (38'),
having a second channel (26) connected with the cylinder (11), wherein in the second channel (26) a second valve (28) is arranged having a second valve closing member (32) assigned to a second valve seat (38),
**characterized in that**
the first valve (27) serves as inlet valve and the second valve (28) serves as outlet valve that are arranged on top of each other, wherein the second valve (28) is arranged above the first valve (27).

2. Pump unit according to claim 1, **characterized in that** the first valve (27) and the second valve (28) are differential pressure-controlled valves.

3. Pump unit according to claim 1 or 2, **characterized in that** the piston (21) is sealed against a cylinder wall (22) of the at least one cylinder (11) and that the second valve (28) is arranged directly adjacent to the cylinder wall (22) at the vertically highest point of cylinder wall (22).

4. Pump unit according to any of the preceding claims, **characterized in that** at least one of the valve closing members (32) is configured seamlessly monolithically of an elastic material.

5. Pump unit according to any of the preceding claims, **characterized in that** at least one of the valve closing members (32) comprises a domed sealing surface (42) facing valve seat (38).

6. Pump unit according to any of the preceding claims, **characterized in that** at least one of the valve seats (38) is a ring-shaped rib-like projection surrounding an inlet opening.

7. Pump unit according to any of the preceding claims, **characterized in that** at least one of the valve closing members (32, 32') comprises an elastic shank section (34) that is held in a support structure (40) through which no flow passes and that is opposite valve seat (38, 38'), and that at least one of the valve closing members (32) comprises a head section (33) from which the elastic shank section (34) extends away.

8. Pump unit according to claim 7, **characterized in that** the shank section (34) is configured in a straight and pressure-elastic manner.

9. Pump unit according to claim 7 or 8, **characterized in that** at least one of the valve closing members (32) is held in a play-free manner between an abutment (36) formed on its support structure (40) and the valve seat (38).

10. Pump unit according to claim 9, **characterized in that** the support structure (40) is arranged in a manner surrounding shank section (34).

11. Pump unit according to claim 10, **characterized in that** the support structure (40) is arranged in the channel (26) and defines a ring-shaped flow cross-section with the wall thereof.

12. Pump unit according to any of the preceding claims, **characterized in that** it comprises two cylinders (11, 12) that are arranged in a horizontally lying manner respectively.

13. Pump unit according to claim 12, **characterized in that** the two cylinders (11, 12) are arranged vertically on top of each other.

14. Pump unit according to any of the preceding claims, **characterized in that** the cylinder (11, 12) comprises a first section (45) and a second section (46), wherein the second section (46) has a diameter (D2) that is larger than the diameter (D1) of first section (45).

15. Pump unit according to claim 14, **characterized in that** the piston (21) is provided with a seal (47, 49) in the first section (45) of cylinder (11, 12) as well as in the second section (46) of cylinder (11, 12) respectively that seals the piston (21) against the cylinder (11, 12).

16. Pump unit according to claim 15, **characterized in that** the first section (45) and the second section (46) of cylinder (11, 12) are at least as long as the maximum pump stroke of piston (21) respectively.

## Revendications

1. Unité de pompe (10) médicale, destinée à l'hydrochirurgie,
comprenant au moins un cylindre (11) avec un piston (21) monté de manière coulissante dans celui-ci,
comprenant une première conduite (25) qui est reliée au cylindre (11) et dans laquelle est disposée une première valve (27) dotée d'un premier élément obturateur de valve (32') associé à un premier siège de valve (38'),
comprenant une deuxième conduite (26) qui est reliée au cylindre (11) et dans laquelle est disposée une deuxième valve (28) dotée d'un deuxième élément obturateur de valve (32) associé à un deuxième siège de valve (38),
**caractérisée en ce que**
la première valve (27) sert de valve d'entrée et la deuxième valve (28) sert de valve de sortie, qui sont disposées l'une au-dessus de l'autre, la deuxième valve (28) étant placée au-dessus de la première valve (27).

2. Unité de pompe selon la revendication 1, **caractérisée en ce que** les valves (27, 28) sont des valves commandées par pression différentielle.

3. Unité de pompe selon la revendication 1 ou 2, **caractérisée en ce que** le piston est étanchéifié vis-à-vis d'une paroi de cylindre (22) du cylindre (11), au nombre d'au moins un, et **en ce que** la deuxième valve (28) est disposée à proximité immédiate de la paroi de cylindre (22), au point verticalement le plus haut de la paroi de cylindre (22).

4. Unité de pompe selon une des revendications précédentes, **caractérisée en ce qu'**au moins un des éléments obturateurs de valve (32) est réalisé d'une seule pièce, sans soudure, à partir d'un matériau élastique.

5. Unité de pompe selon une des revendications précédentes, **caractérisée en ce qu'**au moins un des éléments obturateurs de valve (32) présente une surface d'étanchéité (42) bombée, tournée vers le siège de valve (38).

6. Unité de pompe selon une des revendications précédentes, **caractérisée en ce qu'**au moins un des sièges de valve (38) est une protubérance annulaire en forme de nervure, qui entoure une ouverture d'arrivée.

7. Unité de pompe selon une des revendications précédentes, **caractérisée en ce qu'**au moins un des éléments obturateurs de valve (32, 32') présente une partie de tige (34) élastique avec laquelle il est disposé en étant serti dans une structure de support (40) non traversée par un flux, située à l'opposé du siège de valve (38, 38'), et **en ce qu'**au moins un des éléments obturateurs de valve (32) présente une partie de tête (33) à partir de laquelle s'étend la partie de tige (34) élastique.

8. Unité de pompe selon la revendication 7, **caractérisée en ce que** la partie de tige (34) est réalisée sous une forme rectiligne et de manière élastique à la compression.

9. Unité de pompe selon la revendication 7 ou 8, **caractérisée en ce qu'**au moins un des éléments obturateurs de valve (32) est tenu sans jeu entre une butée (36), réalisée sur sa structure de support (40), et le siège de valve (38).

10. Unité de pompe selon la revendication 9, **caractérisée en ce que** la structure de support (40) est disposée de manière à entourer la partie de tige (34).

11. Unité de pompe selon la revendication 10, **caractérisée en ce que** la structure de support (40) est disposée dans la conduite (26) et définit une section d'écoulement annulaire avec la paroi de celle-ci.

12. Unité de pompe selon une des revendications précédentes, **caractérisée en ce qu'**elle présente deux cylindres (11, 12) qui sont chacun disposés horizontalement.

13. Unité de pompe selon la revendication 12, **caractérisée en ce que** les deux cylindres (11, 12) sont disposés verticalement l'un au-dessus de l'autre.

14. Unité de pompe selon une des revendications précédentes, **caractérisée en ce que** le cylindre (11, 12) présente une première partie (45) et une deuxième partie (46), la deuxième partie (46) ayant un diamètre (D2) qui est supérieur au diamètre (D1) de la première partie (45).

15. Unité de pompe selon la revendication 14, **caractérisée en ce que** le piston (21) est pourvu, aussi bien dans la première partie (45) du cylindre (11, 12) que dans la deuxième partie (46) du cylindre (11, 12), respectivement d'un joint d'étanchéité (47, 49) qui assure l'étanchéité du piston (21) vis-à-vis du cylindre (11, 12).

16. Unité de pompe selon la revendication 15, **caractérisée en ce que** la première partie (45) et la deuxième partie (46) du cylindre (11, 12) ont chacune au moins la même longueur que la course maximale du piston (21).
